# FASCICULE DE BREVET EUROPEEN

(11) **EP 1 296 917 B1**
(45) Date de publication et mention de la délivrance du brevet: **09.07.2008**
(21) Numéro de dépôt: 01929432.1
(22) Date de dépôt: 07.03.2001
(51) Int. Cl.: C07C 17/383, C07C 19/08, G01N 30/72

(54) **PROCEDE POUR L'OBTENTION D'UN HYDROFLUOROALCANE EPURE**
VERFAHREN ZUR HERSTELLUNG VON EINEM GEREINIGTEN HYDROFLUORALKAN
METHOD FOR OBTAINING A PURIFIED HYDROFLUOROALKANE

(30) Priorité: 07.03.2000 FR 0002944
(43) Date de publication de la demande: 02.04.2003
(60) Demande divisionnaire: 06125186.4
(73) Titulaire: SOLVAY (Société Anonyme), 1050 Bruxelles (BE)
(72) Inventeur: BALTHASART, Dominique, B-1120 Bruxelles (BE); ANCIAUX, Charles-Marie, F-21170 Saint-Usage (FR); MAHAUT, Yves, B-1600 Sint-Pieters-Leeuw (BE); KLUG, Roland, 65510 Idstein (DE)
(74) Mandataire: Jacques, Philippe
(86) Numéro de dépôt international: PCT/EP2001/002806
(87) Numéro de publication internationale: WO 2001/066498

(56) Documents cités:
- EP-A- 0 389 334
- EP-A- 0 467 531
- EP-A- 0 472 391
- EP-A- 0 507 458
- EP-A- 0 508 631
- EP-A- 0 526 002
- WO-A-00/14040
- WO-A-94/19301
- WO-A-97/37955
- WO-A-99/50208
- US-A- 4 129 603
- US-A- 5 475 169
- US-A- 5 696 310
- DATABASE WPI Section Ch, Week 199641 Derwent Publications Ltd., London, GB; Class A60, AN 1996-408337 XP002170720 & JP 08 198783 A (CENTRAL GLASS CO LTD), 6 août 1996 (1996-08-06)

## Description

La présente invention a pour objet un procédé pour l'obtention d'un hydrofluoroalcane épuré.

Certains hydrofluoroalcanes comme notamment le 1,1,1,2-tétrafluoroéthane et le 1,1,1,2,3,3,3-heptafluoropropane sont utilisables, en raison de leurs propriétés physiques et de leur toxicologie favorable dans des applications pharmaceutiques, notamment comme gaz propulseur dans des aérosol médicaux.

La production industrielle de tels hydrofluoroalcanes fournit cependant un produit qui contient des impuretés saturées et insaturées. Ces impuretés étant souvent toxiques, des standards sévères sont susceptibles d'être adoptés limitant la teneur, par exemple, en impuretés oléfiniques à moins de 5 ppm en volume (Voir par exemple le projet de standard de la FDA (Food and Drug Administration, Center for Drug and Evaluation and Research, Octobre 1998) concernant la teneur en dites impuretés de 1,1,1,2-tétrafluoroéthane pour des produits MDI).

En conséquence il est nécessaire d'épurer l'hydrofluoroalcane de qualité industrielle afin d'obtenir un produit de qualité pharmaceutique. Il est également nécessaire de disposer de méthodes d'analyse permettant de détecter et d'identifier des traces d'impuretés organiques insaturées et saturées dans l'hydrofluoroalcane. Ceci pose des problèmes notamment lorsque ces impuretés ont un point d'ébullition proche de l'hydrofluoroalcane.

La demande de brevet WO-A-90/8750 concerne un procédé d'épuration de 1,1,1,2-tétrafluoroéthane en impuretés oléfiniques selon lequel on soumet le 1,1,1,2-tétrafluoréthane à une hydrogénation catalytique. Selon ce procédé connu, on observe des teneurs allant jusqu'à 10 ppm en une seule impureté oléfinique, à savoir le 1,1-difluoro-2-chloroéthylène. Selon la demande de brevet, la limite détection de 1,1-difluoro-2-chloroéthylène est de 10 ppm. De plus, ce procédé connu ne permet pas une épuration satisfaisante en toutes les impuretés organiques saturées et insaturées. Notamment, ce procédé connu ne permet pas d'éliminer le 1,1,2,2-tétrafluoroéthane (HFC-134). La présence de quantités substantielles de HFC-134 dans du 1,1,1,2-tétrafluoroéthane pour applications pharmaceutiques n'est pas souhaitable dès lors que sa toxicité a été peu examinée.

La demande de brevet EP 047531 concerne un procédé d'épuration de 1,1,1,2-tétrafluoroéthane selon lequel on soumet 1,1,1,2-tétrafluoroéthane à aux moins deux distillations pour obtenir substantiellement pur 1,1,1,2-tétrafluoroéthane.

Il était dès lors souhaitable de disposer d'un procédé de fabrication efficace permettant d'accéder à du HFC-134a épuré, de qualité pharmaceutique. Il était particulièrement souhaitable de disposer d'un procédé de fabrication permettant une réduction efficace de la teneur en chaque impureté organique individuelle tout en atteignant des teneurs globales en impuretés organiques très basses.

L'invention concerne dès lors un procédé pour l'obtention de 1,1,1,2-tétrafluoroéthane (HFC-134a) de qualité pharmaceutique épuré selon lequel on soumet du HFC-134a comprenant des impuretés organiques à au moins deux distillations, la deuxième distillation étant effectuée à une pression supérieure à celle de la première distillation, et on récupère, en sortie de la deuxième distillation, au moins une fraction constituée de 1,1,1,2-tétrafluoroéthane (HFC-134a) de qualité pharmaceutique épuré en impuretés organiques. Le plus souvent, cette fraction est directement utilisable pour des applications pharmaceutiques. Le procédé selon l'invention n'exclut cependant pas une ou plusieurs étapes de finition supplémentaires.

Dans une première variante du procédé selon l'invention, les impuretés lourdes sont séparées du HFC-134a dans la première distillation et les impuretés légères sont séparées du HFC 134a dans la deuxième distillation.

Dans une deuxième variante du procédé selon l'invention, qui est préférée, les impuretés légères sont séparées du HFC-134a dans la première distillation et les impuretés lourdes sont séparées du HFC-134a dans la deuxième distillation. Dans ce cas, on récupère la fraction constituée de HFC-134a épuré en impuretés organiques en tête de la deuxième distillation.

Par impureté légère on entend désigner une impureté présentant, à la pression de la distillation, un point d'ébullition inférieur à celui du HFC-134a. Par impureté lourde, on entend désigner une impureté présentant, à la pression de la distillation, un point d'ébullition supérieur à celui du HFC-134a.

Il a été trouvé, de manière surprenante, que le procédé selon l'invention permet une épuration extrêmement efficace du HFC-134a en impuretés organiques saturées et insaturées, fournissant ainsi du HFC-134a présentant une basse teneur en lesdites impuretés organiques, notamment en ce qui concerne sa teneur en impuretés saturées et, le cas échéant, en (chloro)fluoroéthènes et (chloro)fluoropropènes. Le procédé selon l'invention s'applique à l'obtention de 1,1,1,2-tétrafluoroéthane de qualité pharmaceutique.

La pression dans la première distillation est généralement inférieure à 10 bar absolus. Souvent elle est d'au plus 9 bar. De préférence elle est d'au plus 8 bar. Généralement la pression dans la première distillation est d'au moins 1 bar. Souvent elle est d'au moins 1,5 bar. De préférence elle est d'au moins 2 bar.

La pression dans la deuxième distillation est généralement supérieure à 10 bar absolus. Souvent elle est d'au moins 12 bar. De préférence elle est d'au moins 15 bar. De manière particulièrement préférée, elle est d'au moins 17 bar. De manière tout particulièrement préférée, elle est d'au moins 19 bar. Généralement la pression dans la deuxième distillation est d'au plus 40 bar. Souvent elle est d'au plus 30 bar. De préférence elle est d'au plus 25 bar.

Dans la présente description, toute référence à la pression correspond à la pression absolue mesurée en tête de la colonne de distillation.

La température à laquelle s'effectue la première ou la deuxième distillation correspond de manière approximative à la température d'ébullition du HFC-134a à la pression choisie pour la distillation respective.

Chacune des deux distillations peut être réalisée dans une ou plusieurs colonnes de distillation. De préférence, on utilisera une seule colonne par distillation.

Les colonnes de distillation utilisables dans le procédé selon l'invention sont connues en elles-mêmes. On peut utiliser, par exemple, des colonnes à plateaux conventionnels ou des colonnes à plateaux de type « dual-flow » ou encore des colonnes à empilage en vrac ou structuré.

Le nombre de plateaux théoriques dans la première distillation est généralement d'au moins 10. Souvent il est d'au moins 15. Un nombre d'au moins 20 donne de bons résultats.

Le nombre de plateaux théoriques dans la deuxième distillation est généralement d'au moins 20. Souvent il est d'au moins 30. Un nombre d'au moins 40 donne de bons résultats.

Le taux de reflux molaire dans la première distillation est généralement d'au moins 50.

Le taux de reflux molaire dans la deuxième distillation est généralement d'au moins 5.

Il a été trouvé que le procédé selon l'invention permet une épuration extrêmement efficace du HFC-134a en impuretés organiques saturées et insaturées, fournissant ainsi du HFC-134a présentant une basse teneur en lesdites impuretés organiques, notamment en ce qui concerne, le cas échéant, sa teneur en (chloro)fluoroéthènes, (chloro)fluoropropènes et en impuretés saturées. En effet le procédé selon l'invention permet l'obtention d'un HFC-134a dans lequel la teneur en chaque impureté organique individuelle a été fortement réduite.

Le HFC-134a obtenu selon le procédé selon l'invention peut présenter une teneur individuelle en chaque impureté organique de moins de 10 ppm molaire. De préférence cette teneur individuelle est d'au plus 8 ppm. De manière particulièrement préférée cette teneur individuelle est d'au plus 5 ppm. Une teneur individuelle d'au plus 2,8 ppm est tout particulièrement préférée. Une teneur individuelle d'au plus 1 ppm est encore plus préférée.

Les teneurs en impuretés dans le HFC-134a obtenu selon le procédé selon l'invention peuvent être déterminées, de manière avantageuse, selon la méthode décrite dans l'exemple 4.

La teneur totale en impuretés organiques dans le HFC-134a obtenu selon le procédé selon l'invention est généralement d'au plus 200 ppm molaire. Souvent cette teneur totale est d'au plus 100 ppm. Plus souvent la teneur totale est d'au plus 50 ppm. De préférence la teneur totale est d'au plus 30 ppm. De manière particulièrement préférée, la teneur totale est d'au plus 25 ppm. Une teneur totale d'au plus 20 ppm est encore plus préférée. La teneur totale peut être même d'au plus 5 ppm.

La teneur totale en impuretés organiques insaturées dans le HFC-134a obtenu selon le procédé selon l'invention est généralement d'au plus 5 ppm molaire. Souvent cette teneur est d'au plus 3 ppm. Plus souvent la teneur est d'au plus 2 ppm. De préférence la teneur est d'au plus 1,8 ppm. De manière particulièrement préférée, la teneur est d'au plus 1,7 ppm.

La teneur en fluoropropènes déterminée dans le 1,1,1,2-tétrafluoroéthane obtenu selon le procédé selon l'invention est généralement moins de 1,4 ppm molaire. Souvent cette teneur est d'au plus 1,2 ppm. Plus souvent, la teneur est d'au plus 1,0 ppm. De préférence la teneur est d'au plus 0,9 ppm. De manière particulièrement préférée, la teneur est d'au plus 0,8 ppm.

La teneur en 1,1,2,2-tétrafluoroéthane dans le 1,1,1,2-tétrafluoroéthane obtenu selon le procédé selon l'invention est généralement moins de 10 ppm molaire. De préférence la teneur est d'au plus 8 ppm. De manière particulièrement préférée, la teneur est d'au plus 5 ppm. De manière encore plus préférée, la teneur est d'au plus 2 ppm.

Le HFC-134a obtenu selon le procédé selon l'inventionpeut-être utilisé comme gaz propulseur dans des aérosols pharmaceutiques ou en industrie électronique.

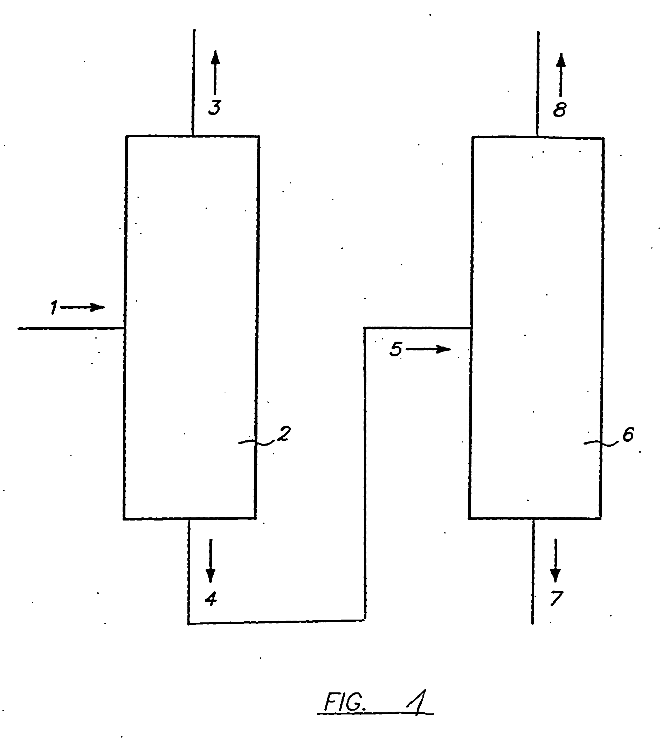

La figure 1 représente un schéma d'installation utilisable pour mettre en oeuvre la variante préférée du procédé d'obtention selon l'invention. Les numéros font référence à la figure 1. Le HFC-134a contenant des impuretés organiques est introduit par la voie (1) dans une première colonne de distillation (2). En tête (3) de cette colonne (2) on obtient une fraction comprenant des impuretés légères (point d'ébullition inférieur mais voisin), et celles qui forment à cette pression un azéotrope à température minimum. En pied (4) de cette colonne (2) on obtient une fraction contenant du HFC-134a avec une teneur réduite en impuretés légères que l'on introduit par la voie (5) dans la deuxième colonne de distillation (6) fonctionnant à pression plus élevée que la colonne (2). On récupère, en pied (7) de la deuxième colonne (6), une fraction enrichie en impuretés lourdes qui présentent un point d'ébullition supérieur par rapport à celui du HFC-134a. En tête (8) de la deuxième colonne (6), on obtient du HFC-134a épuré en impuretés organiques.

Les exemples donnés ci-après entendent illustrer, de manière non limitative, le procédé selon l'invention.

### Exemple 1 (conforme à l'invention)

On a mis en oeuvre une fraction impure de 1,1,1,2-tétrafluoroéthane contenant 57,2 ppm molaire d'impuretés organiques saturées et 10,6 ppm molaire d'impuretés organiques insaturées. On a introduit cette fraction au niveau du 7 ème plateau théorique dans une première colonne de distillation comprenant 20 plateaux théoriques. La pression dans la première colonne était de 6.75 bars abs. On a assuré un taux de reflux de 250. On a soutiré en tête une fraction correspondant à 10 % de l'alimentation et contenant 51,4 ppm molaire d'impuretés organiques saturées et 3,9 ppm molaire d'impuretés organiques insaturées. En pied on a récupéré une fraction constituée de 1,1,1,2-tétrafluoroéthane épuré en impuretés «légères » contenant 41,5 ppm molaire d'impuretés organiques saturées et 34.9 ppm d'impuretés organiques insaturées que l'on a introduit au niveau du 13 ème plateau dans une deuxième colonne de distillation contenant 20 plateaux théoriques. La pression de cette deuxième colonne de distillation était de 19,3 bars absolu. On a assuré un taux de reflux de 14,8. En pied de colonne on a soutiré 10 % de l'alimentation de la première colonne et contenant 320 ppm molaire d'impuretés organiques saturées et 9,8 ppm molaire d'impuretés organiques insaturées En tête de cette colonne on a soutiré une fraction constituée de 1,1,1,2-tétrafluoroéthane épuré en impuretés lourdes contenant 10,7 ppm molaire d'impuretés organiques saturées et 1,3 ppm molaire d'impuretés organiques insaturées.

### Exemple 2 (non conforme à l'invention)

On a mis en oeuvre une fraction impure de 1,1,1,2-tétrafluoroéthane contenant 2750 ppm molaire d'impuretés organiques saturées et 75 ppm molaire d'impuretés organiques insaturées. On a introduit cette fraction au niveau du 14 ème plateau théorique dans une première colonne de distillation comprenant 20 plateaux théoriques. La pression dans la première colonne était de 13 bars abs. On a assuré un taux de reflux de 50. On a soutiré en tête une fraction correspondant à 10% de l'alimentation et contenant 1950 ppm molaire d'impuretés organiques saturées et 150 ppm molaire d'impuretés organiques insaturées. En pied on a récupéré une fraction constituée de 1,1,1,2-tétrafluoroéthane épuré en impuretés «légères» contenant 2950 ppm molaire d'impuretés organiques saturées et 40 ppm d'impuretés organiques insaturées que l'on a introduit au niveau du 14 ème plateau dans une deuxième colonne de distillation contenant 20 plateaux théoriques. La pression de cette deuxième colonne de distillation était de 12 bars absolu. On a assuré un taux de reflux de 12,5. En pied de colonne on a soutiré 10% de l'alimentation de la première colonne et contenant 15500 ppm molaire d'impuretés organiques saturées et 50 ppm molaire d'impuretés organiques insaturées En tête de cette colonne on a soutiré une fraction constituée de 1,1,1,2-tétrafluoroéthane épuré en impuretés lourdes contenant 275 ppm molaire d'impuretés organiques saturées et 31 ppm molaire d'impuretés organiques insaturées.

### Exemple 3 (non conforme à l'invention)

On a mis en oeuvre une fraction impure de 1,1,1,2-tétrafluoroéthane contenant 2950 ppm molaire d'impuretés organiques saturées et 50 ppm molaire d'impuretés organiques insaturées. On a introduit cette fraction au niveau du 14 ème plateau théorique dans une première colonne de distillation comprenant 20 plateaux théoriques. La pression dans la première colonne était de 7,5 bars abs. On a assuré un taux de reflux de 50. On a soutiré en tête une fraction correspondant à 10% de l'alimentation et contenant 1830 ppm molaire d'impuretés organiques saturées et 200 ppm molaire d'impuretés organiques insaturées. En pied on a récupéré une fraction constituée de 1,1,1,2-tétrafluoroéthane épuré en impuretés «légères » contenant 3200 ppm molaire d'impuretés organiques saturées et 32 ppm d'impuretés organiques insaturées que l'on a introduit au niveau du 14 ème plateau dans une deuxième colonne de distillation contenant 20 plateaux théoriques. La pression de cette deuxième colonne de distillation était de 6,5 bars absolu. On a assuré un taux de reflux de 12,5. En pied de colonne on a soutiré 10% de l'alimentation de la première colonne et contenant 27500 ppm molaire d'impuretés organiques saturées et 50 ppm molaire d'impuretés organiques insaturées En tête de cette colonne on a soutiré une fraction constituée de 1,1,1,2-tétrafluoroéthane épuré en impuretés lourdes contenant 200 ppm molaire d'impuretés organiques saturées et 28 ppm molaire d'impuretés organiques insaturées.

### Exemple 4

On a effectué une analyse de 1,1,1,2-tétrafluoroéthane de qualité pharmaceutique obtenu selon un procédé d'obtention conforme à l'invention. Pour ce faire, on a effectué une chromatographie gazeuse sur une colonne de chromatographie gazeuse Rtx®-1 commercialisée par la société RESTEK Corp, comprenant à titre de phase stationnaire 100% de diméthylpolysiloxane réticulé par le procédé Crossbond®. Les dimensions de la colonne étaient 105m x 0.25 mm x 1,0 micron. On a utilisé un programme de température comprenant 2 étapes, la première commençant à -50°C et on a fait monter la température à une vitesse de 1°C/min jusqu'à une température de 0°C. Ensuite, dans une deuxième étape on a fait monter la température à une vitesse de 10°C/min jusqu'à 250°C. La détection a été effectuée par spectrométrie de masse en mode ciblage sur ion caractéristique (selected ion monitoring (SIM)) en utilisant un spectromètre de masse HP 5972 commercialisé par la société Hewlett Packard. On a effectué un étalonnage en mettant en oeuvre un mélange étalon gazeux constitué de 10 ppm de chaque impureté à analyser.

Le tableau ci-après montre la teneur en différentes impuretés du 1,1,1,2-tétrafluoroéthane obtenu selon l'invention et du 1,1,1,2-tétrafluoroéthane brut qui a été mis en oeuvre dans le procédé selon l'invention. La limite de détection de la méthode d'analyse est également indiquée. Cette limite de détection a été validée par un étalonnage statistique sur 5 concentrations de 1 à 10 ppm en chaque impureté individuelle. Les valeurs obtenues ont été corrigées en fonction de la pureté de chacune des impuretés présentes dans le mélange d'étalonnage.

Le tableau reprend en plus les valeurs contenues dans le projet de standard de FDA cité plus haut. Toutes les valeurs sont exprimées en ppm molaire, hormis la valeur «assay » qui est exprimée en pourcent. Un champ vide signifie que l'impureté en question n'a pas été observée.

| **Impureté organique** | **Formule** | **Limite de Détection de la méthode selon l'invention** | **Projet de Standard FDA** | **HFC-134a Brut** | **HFC-134a Invention** |
|---|---|---|---|---|---|
| **HFC-23** | CHF3 | 0.2 | 5 | | |
| **CFC-13** | CC1F3 | 0.3 | 5 | < | |
| **HFC-32** | CH2F2 | 0.2 | 5 | | |
| **HFC-125** | CHF2-CF3 | 0.3 | 5 | 50 | |
| **HFC-143a** | CH3-CF3 | 0.4 | 10 | 124 | |
| **CFC-115** | CCIF2-CF3 | 0.2 | 5 | 0.3 | |
| **HFC-1123** | CHF=CF2 | 0.2 | 5 | | |
| **HFC-1318my-c/t** | CF3-CF=CF-CF3 | 0.7 | 5 | | |
| **HFC-1318my-t/c** (1) | CF3-CF=CF-CF3 | 0.3 | 5 | < | < |
| **HFC-245cb** | CF3-CF2-CH3 | 0.2 | 5 | 0.3 | |
| **HFC-1234yf** | CH2=CF-CF3 | 0.3 | 5 | 1.3 | |
| **HFC-134** | CHF2-CHF2 | 0.4 | 1000 | 2824 | < |
| **HFC-152a** | CH3-CHF2 | 0.2 | 300 | 1.3 | |
| **HFC-217ba** | CF3-CCIF-CF3 | 0.5 | 5* | 2.4 | 0.5 |
| **HFC-161** | CH3-CH2F | 0.2 | 30 | | |
| **HFC-1225ye** | CHF=CF-CF3 | 0.2 | 5 | 1.8 | |
| **HFC-1243zf** | CH2=CH-CF3 | 0.4 | 5 | 7.3 | 0.9 |
| **HFC-1132** | CHF=CHF | 0.4 | 5 | < | |
| **C3H2F4** (2) | | 0.3 | 5* | 11.8 | |
| **HCFC-22** | CHC1F2 | 0.2 | 50 | 0.2 | |
| **HFC-1336mzz** | CF3-CH=CH-CF3 | 0.5 | 5 | | |
| **CFC-12** | CC12F2 | 0.2 | 100 | 0.2 | |
| **HCC-40** | CH3Cl | 0.4 | 5 | 4.0 | |
| **HCFC-124a** | CHF2-CC1F2 | 0.2 | 5 | | |
| **HCFC-124** | CHC1F-CF3 | 0.4 | 100 | 36 | |
| **HCFC-1122** | CHC1=CF2 | 0.3 | 5 | 28 | 0.3 |
| **HCFC-31** | CH2C1F | 0.4 | 5 | 0.4 | |
| **CFC-114** | CC1F2-CC1F2 | 0.1 | 5 | | |
| **CFC-114a** | CC12F-CF3 | 0.1 | 25 | 19 | |
| **HFC-152** | CH2F-CH2F | 0.5 | 5 | | |
| **HCFC-1122a c/t** (3) | CHF=CCIF | 0.3 | 5* | 4.6 | |
| **HCFC-1122a** | CHF=CC1F | 0.3 | 5* | < | |
| **c/t(3)** | | | | | |
| **HCFC-133a** | CH2Cl-CF3 | 0.4 | 5 | 0.4 | |
| **HCFC-1131trans** | CHCl=CHF | 0.5 | 5* | 10.3 | 0.2 |
| **HCFC-1131cis** | CHCl=CHF | 0.5 | 5* | | |
| **CFC-12B1** | CCIBrF2 | 0.2 | 5 | | |
| **CFC-1112a** | CF2=CCl2 | 0.3 | 5 | | |
| **HCFC-123** | CHCl2-CF3 | 0.4 | 5 | | |
| **CFC-11** | CCl3F | 0.3 | 5 | | |
| **HCFC-123a** | CHClF-CClF2 | 0.3 | 5 | | |
| **HCFC-1121** c/t | CHCl=CClF | 0.2 | 5 | | |
| **HCC-30** | CH2Cl2 | 0.6 | 5* | < | |
| **HCFC-132b** | CClF2-CH2Cl | 0.6 | 5 | | |
| **CFC-113** | CClF2-CCl2F | 0.4 | 5 | | |
| **HCC-1120** | CHCl=CCl2 | 0.8 | 5* | < | < |
| | | | | | |
| | | | | | |
| **Somme d'impuretés organiques** | | | **1000** | **3127.6** | **1.9** |
| **Somme d'oléfines** | | | **5** | **65.1** | **1.4** |
| **Assay** | | | **99.9%** | | **100%** |

| | | | | | |
|---|---|---|---|---|---|
| < inférieur à la limite de détection Les impuretés dont la teneur est inférieure à la limite de détection n'ont pas été prises en compte pour le calcul des sommes d'impuretés. (1) valeur estimée (2) valeur calculée en prenant en compte le facteur de réponse du HFC-1234yf (3) isomères E/Z, valeur calculée en prenant en compte le facteur de réponse du HCFC-1122 * Composé non cité individuellement dans le projet de standard FDA. | | | | | |

Il apparaît que le 1,1,1,2-tétrafluoroéthane, obtenu conformément au procédé selon l'invention présente une teneur en impuretés organiques, extrêmement basse et largement inférieure aux valeurs du projet de standard FDA. De plus, la teneur d'aucune impureté organique individuelle ne dépasse 1 ppm molaire.

Il apparaît également que la méthode d'analyse permet de détecter et d'identifier, avec une sensibilité extrêmement élevée toutes les impuretés incluses dans le projet de standard de la FDA.

## Revendications

1. - Procédé pour l'obtention de 1,1,1,2-tétrafluoroéthane de qualité pharmaceutique épuré selon lequel on soumet du 1,1,1,2-tétrafluoroéthane comprenant des impuretés organiques à au moins deux distillations, la deuxième distillation étant effectuée à une pression supérieure à celle de la première distillation, et on récupère, en sortie de la deuxième distillation, au moins une fraction constituée de 1,1,1,2-tétrafluoroéthané de qualité pharmaceutique, épuré en impuretés organiques.

2. - Procédé selon la revendication 1 dans lequel la pression dans la première distillation est inférieure à 10 bar ét la pression dans la deuxième distillation est d'au moins 10 bar.

3. - Procédé selon la revendication 1 ou 2 dans lequel la pression dans la première distillation est d'au plus 9 bar.

4. - Procédé selon la revendication 3 dans lequel la pression dans la première distillation est d'au plus 8 bar.

5. - Procédé selon l'une quelconque des revendications 1 à 4 dans lequel la pression dans la deuxième distillation est d'au moins 15 bar.

6. - Procédé selon la revendication 5 dans lequel la pression dans la deuxième distillation est d'au moins 19 bar.

7. - Procédé selon l'une quelconque des revendications 1 à 6 dans lequel on récupère la fraction constituée de 1,1,1,2-tétrafluoroéthane épuré en impuretés organiques en tête de la deuxième distillation.

## Claims

1. Process for the production of purified 1,1,1,2-tetrafluoroethane of pharmaceutical quality, according to which 1,1,1,2-tetrafluoroethane, comprising organic impurities, is subjected to at least two distillations, the second distillation being carried out at a pressure greater than that of the first distillation, and at least one fraction composed of 1,1,1,2-tetrafluoroethane of pharmaceutical quality purified from organic impurities is recovered at the outlet of the second distillation.

2. Process according to Claim 1, in which the pressure in the first distillation is less than 10 bar and the pressure in the second distillation is at least 10 bar.

3. Process according to Claim 1 or 2, in which the pressure in the first distillation is at most 9 bar.

4. Process according to Claim 3, in which the pressure in the first distillation is at most 8 bar.

5. Process according to any one of Claims 1 to 4, in which the pressure in the second distillation is at least 15 bar.

6. Process according to Claim 5, in which the pressure in the second distillation is at least 19 bar.

7. Process according to any one of Claims 1 to 6, in which the fraction composed of 1,1,1,2-tetrafluoroethane purified from organic impurities is recovered at the top of the second distillation.

## Patentansprüche

1. Verfahren zur Herstellung von gereinigtem 1,1,1,2-Tetrafluorethan pharmazeutischer Qualität, bei dem man organische Verunreinigungen enthaltendes 1,1,1,2-Tetrafluorethan mindestens zwei Destillationen unterwirft, wobei die zweite Destillation bei einem über dem Druck der ersten Destillation liegenden Druck durchgeführt wird und am Ausgang der zweiten Destillation mindestens eine Fraktion aus von organischen Verunreinigungen befreitem 1,1,1,2-Tetrafluorethan pharmazeutischer Qualität gewonnen wird.

2. Verfahren nach Anspruch 1, bei dem der Druck bei der ersten Destillation unter 10 bar liegt und der Druck bei der zweiten Destillation mindestens 10 bar beträgt.

3. Verfahren nach Anspruch 1 oder 2, bei dem der Druck bei der ersten Destillation höchstens 9 bar beträgt.

4. Verfahren nach Anspruch 3, bei dem der Druck bei der ersten Destillation höchstens 8 bar beträgt.

5. Verfahren nach einem der Ansprüche 1 bis 4, bei dem der Druck bei der zweiten Destillation mindestens 15 bar beträgt.

6. Verfahren nach Anspruch 5, bei dem der Druck bei der zweiten Destillation mindestens 19 bar beträgt.

7. Verfahren nach einem der Ansprüche 1 bis 6, bei dem man die Fraktion aus von organischen Verunreinigungen befreitem 1,1,1,2-Tetrafluorethan am Kopf der zweiten Destillation gewinnt.
